# EUROPÄISCHE PATENTSCHRIFT

(11) **EP 2 760 978 B1**
(45) Veröffentlichungstag und Bekanntmachung des Hinweises auf die Patenterteilung: **30.10.2019**
(21) Anmeldenummer: 12766425.8
(22) Anmeldetag: 21.09.2012
(51) Int. Cl.: C10L 3/08, C02F 3/28, C12P 5/02

(54) **VERFAHREN ZUR ERZEUGUNG VON BIOGAS**
PROCESS FOR PRODUCTION OF BIOGAS
PROCÉDÉ DE PRODUCTION DE BIOGAZ

(30) Priorität: 28.09.2011 DE 102011114441; 22.03.2012 DE 102012005636
(43) Veröffentlichungstag der Anmeldung: 06.08.2014
(73) Patentinhaber: Rheinkalk GmbH, 42489 Wülfrath (DE)
(72) Erfinder: PICKBRENNER, Arnd, 42489 Wülfrath (DE); SAURE, Heiko, 58285 Gevelsberg (DE); PUST, Christopher, 40625 Düsseldorf (DE); SPICKER, Volker, 42489 Wülfrath (DE)
(74) Vertreter: Cohausz & Florack
(86) Internationale Anmeldenummer: PCT/EP2012/068669
(87) Internationale Veröffentlichungsnummer: WO 2013/045368

(56) Entgegenhaltungen:
- EP-A1- 0 218 896
- DE-A1-102007 043 751
- FR-A1- 2 924 441
- US-A1- 2009 107 913

## Beschreibung

Die Erfindung betrifft ein Verfahren zur Erzeugung von Biogas aus einem biologisch abbaubaren Substrat unter anaeroben Bedingungen. Die Erfindung betrifft ferner die Verwendung von kalkstämmigen Materialien zur Stabilisierung des erfindungsgemäßen Verfahrens.

Aufgrund der zunehmenden Verknappung der Rohstoffe für die Energiegewinnung besteht ein weltweit steigendes Bedürfnis für die Erzeugung von Energie aus erneuerbaren Rohstoffen. Aufgrund der kontinuierlichen Verfügbarkeit unabhängig von kurzfristigen Schwankungen von Wind oder Sonneneinstrahlung trägt die Bioenergie und damit auch Biogas sinnvoll dazu bei, eine Ergänzung im Energiemix der erneuerbaren Energieträger einzunehmen.

Die Erzeugung von thermisch nutzbaren Gasgemischen aus biologisch abbaubaren Substraten (Biogasgewinnung) stellt ein technisch relativ einfaches Verfahren zur Umwandlung von Biomasse in Energie dar. Der biochemische Abbau organischer Substanzen durch Mikroorganismen unter anaeroben Bedingungen (Fermentation) ist hinlänglich bekannt.

Biogas selbst ist vor der Aufbereitung eine meist wassergesättigte brennbare Gasmischung mit den Hauptkomponenten Methan (CH₄) und Kohlendioxid (CO₂). In geringen Mengen sind meist auch Stickstoff (N₂), Sauerstoff (O₂), Schwefelwasserstoff (H₂S), Wasserstoff (H₂) und Ammoniak (NH₃) enthalten. Für die Verwertung des Biogases ist der Methananteil am wichtigsten, da er, als oxidierbare Verbindung, bei der Verbrennung Energie freisetzt.

Zur Erhöhung der Wirtschaftlichkeit bei der Biogasproduktion wird im Allgemeinen angestrebt, die eingesetzte Biomasse möglichst vollständig umzusetzen. Ferner ist es erwünscht die Raumbelastung (zugesetzte Masse an organischer Trockensubstanz pro Fermentervolumen- und Zeiteinheit) zu maximieren. Gleichzeitig werden möglichst energiereiche Substrate eingesetzt.

Zu hohe Raumbelastungen können aber leicht zu einem Ungleichgewicht des mehrstufigen Fermentationsprozesses führen. Eine hierdurch hervorgerufene Versauerung des Fermenters führt zu einer Hemmung der methanogenen Mikroorganismen. Im Extremfall kann dies zu einem völligen Erliegen des Prozesses führen. Um derartige Versauerungsprozesse aufzufangen ist es üblich, im Bedarfsfall die Substratzufuhr zu verringern. Ist diese Vorgehensweise nicht erfolgreich, so ist ein kompletter Austausch des Reaktorinhalts erforderlich. Der darauf folgende Anfahrbetrieb des Fermentationsprozesses bis zum Erreichen der maximalen Gasausbeute kann bis zu zwölf Wochen in Anspruch nehmen. Die Minderung der Methanerzeugung bzw. der vollständige Ausfall mit nachfolgendem Anfahrbetrieb zieht direkt eine Ertragsminderung nach sich.

Ein weiteres übliches Verfahren zur Prozessstabilisierung besteht darin der Substratversauerung durch Zugabe neutralisierender Additive entgegenzutreten. Als besonders geeignet für diesen Zweck haben sich kalkstämmige Produkte erwiesen. So beschreibt die Druckschrift JP 632708 die anaerobe fermentative Behandlung einer Mischung aus flüssigen Sojabohnenrückständen und Tonerde, wobei eine Kombination aus Zeolithen und Branntkalk (CaO) zur Verbesserung der Fermentation beigegeben wird.

Die Verwendung kalkstämmiger Produkte zur Stabilisierung der Biogasproduktion ist auch aus der Druckschrift
DE 100 34 279 A1 bekannt. Diese Druckschrift beschreibt ein Verfahren zur Regelung einer Biogasanlage, in dem mittels einer Messeinrichtung für die Methanisierung wichtige Parameter des Sickersafts, z.B. pH-Wert, gemessen werden. Es wird beschrieben, dass bei Absinken des pH-Werts unter einen bestimmten Wert Calciumhydroxid oder Kalkmilch zugesetzt werden, so dass der pH-Wert wieder auf einen gewünschten Wert ansteigt. Die EP 2 090 660 A1 beschreibt ein Verfahren zur Herstellung von Biogas unter Zugabe von Carbokalk.

Nachteilig an den bekannten Verfahren ist, dass die zugesetzten kalkstämmigen Produkte dazu neigen, im Bioreaktor zu sedimentieren, was zu einer Verringerung ihrer Wirksamkeit führt.

EP 0 218 896 A1 offenbart ein Verfahren zur Erzeugung von Biogas aus einem biologisch abbaubaren Substrat unter im Wesentlichen anaeroben Bedingungen, umfassend folgende Schritte: das Einbringen des Substrats in einen Reaktionsraum; Zugabe eines kalkstämmigen Materials, bestehend aus Kalkmilch zu dem Substrat, in Zerkleinerungsvorrichtung; und biologischer Abbau des Substrats, wobei das Kalkmilch, das zur Stabilisierung des pH-Werts eingesetzt wird, einen Teilchendurchmesser von unter 0,4 µm aufweist.

Die der Erfindung zugrunde liegende Aufgabe besteht darin, ein Verfahren der eingangs genannten Art bereitzustellen, mit dem der pH-Wert des biologisch abbaubaren Substrats mittels kalkstämmiger Produkte während des biologischen Abbaus zuverlässig stabilisiert werden kann. So soll die Zugabe kalkstämmiger Produkte zur Aufrechterhaltung der erforderlichen Pufferkapazität des Fermenterinhalts führen. Insbesondere soll mit dem erfindungsgemäßen Verfahren die stabilisierende Wirkung kalkstämmiger Produkte möglichst gut ausgenutzt werden.

Diese Aufgabe wird erfindungsgemäß gelöst durch ein Verfahren zur Erzeugung von Biogas aus einem biologisch abbaubaren Substrat unter im Wesentlichen anaeroben Bedingungen, umfassend die Schritte des Einbringens des Substrats in einen Reaktionsraum, der Zugabe eines kalkstämmigen Materials ausgewählt aus der Gruppe bestehend aus Kalkhydrat, Kalkmilch, Kalksteinmehl, Branntkalk, Dolomitsteinmehl, Dolomithalbbrannt, Dolomitkalkhydrat, Dolomithalbhydrat und/oder Natriumhydrogencarbonat, mit einem Teilchendurchmesser d₉₇ von 1 bis 70 µm und/oder einem Teilchendurchmesser d₅₀ von 1 bis 8 10 µm, sowie des biologischen Substratabbaus unter Bildung von Biogas.

Das erfindungsgemäße Verfahren zeichnet sich dadurch aus, dass feinteilige kalkstämmige Materialien als Additive zur Stabilisierung des Fermentationsprozesses bei der Biogasherstellung eingesetzt werden. Überraschend wurde gefunden, dass derartige Additive keine bzw. nur geringe Sedimentationsneigung im Fermenter zeigen. Somit kann in dem erfindungsgemäßen Verfahren der pH-Wert während des Fermentationsprozesses zuverlässig stabilisiert werden. Dies ermöglicht den Einsatz hochenergetischer Substrate bei gleichzeitig hoher Raumbelastung.

Der Einsatz des kalkstämmigen Materials im erfindungsgemäßen Verfahren zeichnet sich ferner dadurch aus, dass er die Erzeugung von Biogas mit einem hohen Methananteil ermöglicht. Darüber hinaus weisen kalkstämmige Materialien grundsätzlich den Vorteil auf die als Substrate verwendeten organischen Substanzen zu hygienisieren. Dies ist insbesondere bei der Verwendung von landwirtschaftlichen Abfällen vorteilhaft.

Als kalkstämmiges Material können in dem erfindungsgemäßen Verfahren die verschiedensten Substanzen eingesetzt werden. Praktische Versuche haben ergeben, dass mit Kalkhydrat, Kalkmilch, Kalksteinmehl, Branntkalk, Dolomitsteinmehl, Dolomithalbbrannt, Dolomitkalkhydrat, Dolomithalbhydrat und/oder Natriumhydrogencarbonat besonders gute Ergebnisse erzielt werden.

Erfindungsgemäß weist das kalkstämmige Material einen mittleren Teilchendurchmesser d₉₇ von 1 bis 70 µm auf. Es wurde gefunden, dass die Einstellung des Teilchendurchmessers d₉₇ auf Werte zwischen 1 bis 70 µm ausreicht, um eine Sedimentation im Bioreaktor in ausreichendem Maße zu verhindern.

Besonders gute Ergebnisse werden mit kalkstämmigen Materialien erzielt, die einen Teilchendurchmesser d₉₇ von 1 bis 50 µm, vorzugsweise von 8 bis 40 µm, noch bevorzugter von 6 bis 30 pm, insbesondere von 4 bis 15 µm, und/oder einen Teilchendurchmesser d₅₀ von 1 bis 8 µm aufweisen.

Gute Ergebnisse werden auch mit kalkstämmigen Materialien erzielt, die einen Teilchendurchmesser d₉₇ von weniger als 50 µm, vorzugsweise von weniger als 25 µm und insbesondere von weniger als 15 µm aufweisen.

Durch die Additivzugabe kann im erfindungsgemäßen Verfahren der pH-Wert der eingesetzten Substrate während des biologischen Abbaus verlässlich stabilisiert werden. So kann einer Versauerung des Substrats beispielsweise bei erhöhter Raumbelastung und/oder hoher Energiedichte der eingesetzten Substrate wirkungsvoll entgegengetreten werden.

Der Soll-pH-Wert einer Anlage hängt vom Anlagentyp, den Anlagen- und Prozessparametern sowie den eingesetzten Substraten und mikrobiellen Kulturen ab und kann vom Fachmann durch Versuche ermittelt werden. Es hat sich gezeigt, dass üblicherweise mit pH-Werten zwischen 6,5 und 7,5 die besten Ergebnisse erzielt werden.

Gemäß einer bevorzugten Ausgestaltung der Erfindung findet die Zugabe des kalkstämmigen Materials bedarfsabhängig statt, um einer drohenden Versauerung des Substrats entgegenzutreten. In dieser Ausführungsform erfolgt die Zugabe des kalkstämmigen Materials zweckmäßigerweise dann, wenn der pH-Wert des Substrats auf Werte unterhalb von 7,5, vorzugsweise unter 7,0, fällt.

Besonders gute Ergebnisse werden erzielt, wenn die Zugabe des kalkstämmigen Materials dann erfolgt, wenn der gemessene pH-Wert des Substrats höchstens 0,5, vorzugsweise höchstens 0,2, vom jeweiligen Soll-pH-Wert für das Substrat abweicht.

In anderen Fällen kann es wiederum zweckmäßig sein, die Zugabe des kalkstämmigen Materials präventiv zur Stabilisierung des biologischen Substrats vorzunehmen. Eine solche Vorgehensweise, bei der die Zugabe des kalkstämmigen Materials unabhängig vom pH-Wert des Substrats erfolgen kann, ist insbesondere dann zweckmäßig, wenn eine ständige Überwachung der Verfahrensparameter im Bioreaktor vermieden werden soll. Bei dieser Verfahrensvariante wird ausgenutzt, dass durch die Zugabe des kalkstämmigen Materials die Pufferkapazität des Substrats erhöht und somit einer Versauerung vorgebeugt wird.

Zweckmäßigerweise erfolgt die Zugabe des kalkstämmigen Materials bei einer präventiven Zugabe bereits zu Beginn der Biogaserzeugung. Auf diese Weise kann einer Versauerung des Substrats während des gesamten Fermentationsprozesses wirkungsvoll vorgebeugt werden.

Die Menge, in der das kalkstämmige Material zugegeben wird, kann erfindungsgemäß in weiten Bereichen variieren. Zweckmäßigerweise wird die Zugabemenge in Abhängigkeit von der Energiedichte des eingesetzten biologisch abbaubaren Substrats sowie unter Berücksichtigung der jeweiligen Raumbelastung im Bioreaktor eingestellt. Praktische Versuche haben ergeben, dass es in den meisten Fällen sinnvoll ist das kalkstämmige Material in einer Menge von 0,0001 bis 0,05 mol/l, vorzugsweise von 0,001 bis 0,02 mol/l, und insbesondere von 0,005 bis 0,01 mol/l, jeweils bezogen auf das Gesamtvolumen des Fermenterinhalts, zuzugeben.

Soll durch die Zugabe des kalkstämmigen Materials einer drohenden bzw. bereits eingesetzten Versauerung im Bioreaktor entgegengetreten werden, so wird das kalkstämmige Material vorzugsweise in einer solchen Menge zugegeben, dass sich der pH-Wert des Substrats auf einen Wert von 6 bis 8, vorzugsweise von 6,3 bis 7,8, und insbesondere von 6,8 bis 7,5 einstellt. Bei dieser Vorgehensweise ist zu beachten, dass die Einstellung des pH-Werts im Substrat in Abhängigkeit von dem jeweils eingesetzten kalkstämmigen Material, dessen Partikelgröße sowie der Durchmischung im Bioreaktor unterschiedlich lang dauern kann. So hat sich in praktischen Versuchen gezeigt, dass die pH-Wert regulierende Wirkung von Dolomithalbbrannt mit Verzögerung eintritt. Dieser verzögerte Wirkungseintritt ist vorteilhaft, da bedingt durch die Größe des Fermenters und des niedrigen spezifischen Energieeintrags der Rührorgane die Mischzeiten üblicherweise recht hoch sind. Der verzögerte Wirkungseintritt des Dolomithalbbrannts erlaubt daher eine gute Verteilung vor dem Einsetzen der pH-Wert regulierenden Wirkung. Dies führt zu einer besonders guten Ausnutzung der stabilisierenden Wirkung des Dolomithalbbrannts.

Soll mit dem kalkstämmigen Material präventiv die Pufferkapazität des Substrats erhöht werden, so wird das kalkstämmige Material vorzugsweise in einer solchen Menge zugegeben, dass sich die Pufferkapazität, ausgedrückt als Konzentration CaCO₃ pro Liter Reaktionsraum, auf einen Wert von mehr als 5 g/l, vorzugsweise von mehr als 10 g/l, noch bevorzugter von mehr als 15 g/l, und insbesondere von 15 bis 20 g/l einstellt.

Der biologische Abbau der Substrate im erfindungsgemäßen Verfahren, kann auf an sich bekannte Art und Weise erfolgen. So wird der biologische Abbau üblicherweise folgende Schritte umfassen:
- Aufspaltung von im Substrat enthaltenen Kohlenhydraten, Fetten und/oder Eiweißen zu Spaltprodukten umfassend Zucker, Fettsäuren, Aminosäuren und/oder Basen;
- Fermentation der Spaltprodukte zu Vergärungsprodukten umfassend Carbonsäuren, Gase und/oder Alkohole;
- Bildung von methanogenen Substraten umfassend Essigsäure, Wasserstoff und/oder Kohlendioxid;
- Bildung von Biogas umfassend Methan und Kohlendioxid.

Innerhalb dieses Reaktionsschemas kann die Aufspaltung von im Substrat enthaltenen Kohlenhydraten, Fetten und/oder Eiweißen durch hydrolytische Bakterien, die Fermentation der Spaltprodukte zu Vergärungsprodukten durch fermentative Bakterien, die Bildung von methanogenen Substraten durch acetogene Bakterien und die Bildung von Biogas durch methanogene Bakterien erfolgen.

Da die Bakterien der einzelnen Abbaustufen unterschiedliche Anforderungen an ihren Lebensraum stellen, kann es erfindungsgemäß zweckmäßig sein, den biologischen Abbau in einem mehrstufigen System durchzuführen. Auf diese Weise kann den Bedürfnissen der Bakterien der einzelnen Abbaustufen besonders gut Rechnung getragen und eine besonders hohe Biogasausbeute erzielt werden.

Es ist erfindungsgemäß jedoch auch möglich, die einzelnen Schritte des biologischen Abbaus in demselben Reaktionsraum stattfinden zu lassen. So ist es erfindungsgemäß bevorzugt mindestens zwei, vorzugsweise drei, noch bevorzugter alle vier Schritte des biologischen Abbaus in demselben Reaktionsraum stattfinden zu lassen. Diese Reaktionsführung hat den Vorteil einen besonders geringen technischen Aufwand zu erfordern. Bei dieser Verfahrensvariante ist es ferner von Vorteil im Hinblick auf das von den einzelnen Bakterien benötigte Milieu einen Kompromiss zu finden. Besonders relevant ist der im Reaktionsraum eingestellte pH-Wert.

In diesem Zusammenhang wurde gefunden, dass die Methanbakterien am empfindlichsten auf Prozessstörungen reagieren und sich gleichzeitig am langsamsten vermehren. Aus diesem Grund ist es erfindungsgemäß bevorzugt, die Milieueinstellung im Bioreaktor an den Anforderungen dieser Mikroorganismen zu orientieren. Zu diesem Zweck hat es sich als sinnvoll erwiesen, den pH-Wert des Substrats im Bioreaktor auf einen Wert von 6,8 bis 7,5 einzustellen. Ein derartiger pH-Wert ist auch für die Aktivität der acetogenen Bakterien von Vorteil.

Um eine möglichst hohe Aktivität insbesondere der Methanbakterien zu gewährleisten hat es sich darüber hinaus als günstig erwiesen, wenn ein biologisch abbaubares Substrat mit einem C:N:P:S-Verhältnis von etwa
600 : 15 : 5: 1 eingesetzt wird. Gute Ergebnisse werden auch mit einem biologisch abbaubarem Substrat mit einem C:N-Verhältnis von 10 bis 30 erzielt. Vorteilhaft für den Abbauprozess ist ferner die Verwendung eines Substrats mit einer möglichst geringen Hemmstoffkonzentration.

Als ebenfalls günstig im Hinblick auf die bakterielle Aktivität hat sich die Einstellung der Reaktionstemperatur im Fermenter auf einen Wert von 28 bis 58°C, vorzugsweise von 32 bis 42°C, insbesondere bei mesophil betriebenen Biogasanlagen, erwiesen.

Ein weiterer wichtiger Parameter für das erfindungsgemäße Verfahren ist die Raumbelastung des Bioreaktors. Unter Raumbelastung wird erfindungsgemäß die zugesetzte Masse an organischer Trockensubstanz der eingesetzten Substrate pro Fermentervolumen- und Zeiteinheit verstanden. Die Raumbelastung des Bioreaktors kann im erfindungsgemäßen Verfahren in weiten Bereichen schwanken. Vorteilhafterweise wird die Raumbelastung unter Berücksichtigung der verwendeten Anlagentechnik, der Energiedichte der eingesetzten Substrate und der Art und Menge des eingesetzten kalkstämmigen Materials eingestellt.

Praktische Versuche haben gezeigt, dass üblicherweise mit Raumbelastungen in einer Höhe von 0,5 bis 5 kg oTS/m³ Tag, vorzugsweise 1 bis 3 kg oTS/m³ Tag gute Ergebnisse erzielt werden können. Die Raumbelastung wird zweckmäßigerweise möglichst hoch gewählt, um eine hohe Gasausbeute zu erzielen und somit das erfindungsgemäße Verfahren wirtschaftlich zu betreiben.

Das kalkstämmige Material wird zweckmäßigerweise in einer Menge von 0,0001 bis 0,05 mol/l, vorzugsweise von 0,001 bis 0,02 mol/l, und insbesondere von 0,005 bis 0,01 mol/l, jeweils bezogen auf das Gesamtvolumen des Fermenterinhalts, zugegeben.

Das erfindungsgemäße Verfahren kann in einem herkömmlichen Bioreaktor, häufig als Fermenter bezeichnet, durchgeführt werden. Man unterscheidet Bioreaktoren üblicherweise nach Bauweise und Funktionsweise. Jeder Bioreaktor kann drei Phasen verarbeiten: fest (Biomasse), flüssig (Nährmedium) und gasförmig (z. B. Luft, Sauerstoff, Kohlendioxid, Stickstoff, Methan).

Die Auswahl des jeweils geeigneten Bioreaktors hängt zweckmäßigerweise von den Anforderungen an den biologischen Prozess ab. So erhöht zum Beispiel eine effektive Rührerleistung mit hoher Umdrehungszahl die Homogenität des Mediums im Reaktor und den Sauerstoffeintrag. Bei Bioreaktoren ist ein zu starkes Durchmischen vorzugsweise zu vermeiden, um die Lebensgemeinschaft der Bakterien zu erhalten.

Je nach Art der Befüllung unterscheidet man:
- Batch-Prozesse, bei denen der Reaktor einmal komplett befüllt wird und nach Ablauf einer bestimmten Zeitspanne ("Reaktionszeit" oder "Wachstumszeit") wieder komplett entleert wird.
- Fed-Batch-Prozesse, bei denen der Füllstand anfangs unterhalb der maximalen Kapazität des Reaktors liegt und der dann langsam bis zur maximalen Auslastung mit Nährmedium erhöht wird. Dann wird der Reaktor wieder komplett entleert.
- Kontinuierliche Prozesse, bei denen ein steter Zulauf an Nährmedium und ein gleichgroßer Ablauf an "Brühe" (Gemisch aus Nährmedium, Produkt, Biomasse etc.) den Füllstand auf einem konstanten Niveau halten.

Um eine optimale Produktausbeute zu erreichen, kann es zweckmäßig sein die Bedingungen im Inneren der Apparatur mit Hilfe von Sensoren zu überwachen. Mittels Sonden kann somit der pH-Wert, der Sauerstoff-Gehalt, der Kohlendioxid-Gehalt in der Abluft, Temperatur und Schaumentwicklung gemessen werden. Ferner findet vorzugsweise eine ständige Überwachung der Biogasmenge und dessen Zusammensetzung (Gehalt an Methan, CO₂, O₂, SO₂) statt.

Als biologisch abbaubares Substrat können erfindungsgemäß die verschiedensten Substanzen eingesetzt werden. So eignen sich als Ausgangsstoffe für die technische Produktion von Biogas beispielsweise:
- vergärbare, biomassehaltige Reststoffe wie Klärschlamm, Bioabfall oder Speisereste
- Wirtschaftsdünger (Gülle, Mist)
- bisher nicht genutzte Pflanzen bzw. Pflanzenteile (z. B. Zwischenfrüchte, Kleegras im Biolandbau)
- gezielt angebaute Energiepflanzen (Nachwachsende Rohstoffe).

Dabei stellt die Landwirtschaft mit den drei letztgenannten Möglichkeiten das größte Potenzial für die Produktion von Biogas. Bis auf die letzte Möglichkeit handelt es sich dabei um prinzipiell kostenlose Ausgangsstoffe (abgesehen von Transport- und sonstigen Nebenkosten). Erfindungsgemäß besonders geeignete Substrate sind die nachwachsenden Rohstoffe. Durch den Einsatz von Gülle und Stallmist kann der ebenfalls sehr wichtige Bedarf an Spurenelementen zudem meist gedeckt werden.

Ein weiterer Gegenstand der Erfindung ist die Verwendung eines kalkstämmigen Materials gemäß Anspruch 18.

Erfindungsgemäß bevorzugt wird das kalkstämmige Material zur Regulierung des pH-Werts auf einen Wert von 6,3 bis 8,0, vorzugsweise von 6,8 bis 7,5 verwendet.

Ebenfalls bevorzugt ist es, das kalkstämmige Material zur Erhöhung der Pufferkapazität des organischen Substrats zu verwenden.

Vorteilhafterweise wir die Pufferkapazität, ausgedrückt als Konzentration CaCO₃ pro Liter Reaktionsraum, auf einen Wert von mehr als 10 g/l, vorzugsweise mehr als 15 g/l und insbesondere von 15 bis 20 g/l erhöht.

Gemäß einer weiteren bevorzugten Ausführungsform der Erfindung wird das kalkstämmige Material als Additiv zur Herstellung von Biogas mit einem erhöhten Methananteil verwendet. So kann das Biogas einen Methananteil von mehr als 68 Vol.% Methan, vorzugsweise von 50 bis 75 Vol.% Methan, vorzugsweise von 60 bis 75 Vol.% Methan und insbesondere von 65 bis 70 Vol%, aufweisen.

Das mit dem erfindungsgemäßen Verfahren hergestellte Biogas eignet sich nach entsprechender Aufbereitung hervorragend zum Einsatz in Kraft-/Wärme-Kopplungsanlagen, wie insbesondere Blockheizkraftwerken. Da eine Aufbereitung auf Erdgasqualität sinnvoll ist, ist es vorteilhaft zu diesem Zweck den CO₂-Anteil weitestgehend zu entfernen. Das hierbei erhaltene sogenannte Biomethan oder Bioerdgas kann auf 200 bis 300 bar verdichtet werden, um dann in geeigneten Kraftfahrzeugen genutzt werden zu können.

Im Folgenden wird die Erfindung anhand von einer lediglich Ausführungsbeispiele darstellenden Zeichnung näher erläutert.

Es zeigen:
- Fig.1:: die Pufferkapazität bei stoffmengenbezogener Additivdosierung in einem diskontinuierlichen Fermentationsversuch,
- Fig.2:: die Biogasproduktion bei stoffmengenbezogener Additivdosierung in einem diskontinuierlichen Fermentationsversuch,
- Fig.3:: die Methangasproduktion bei stoffmengenbezogener Additivdosierung in einem diskontinuierlichen Fermentationsversuch,
- Fig.4:: die Biogasproduktion bei pH-Wert orientierter Additivdosierung in einem diskontinuierlichen Fermentationsversuch,
- Fig.5:: die Methangasproduktion bei pH-Wert orientierter Additivdosierung in einem diskontinuierlichen Fermentationsversuch,
- Fig.6:: das Verhältnis von Methangasvolumen zu Faulraumbelastung in einem kontinuierlichen Fermentationsversuch bei gleichbleibend hoher Faulraumbelastung nach Übersäuerung,
- Fig.7:: das Verhältnis von Methangasvolumen zu Faulraumbelastung in einem kontinuierlichen Fermentationsversuch bei reduzierter Faulraumbelastung nach Übersäuerung,
- Fig.8:: das Verhältnis von Methangasvolumen zu Faulraumbelastung in einem kontinuierlichen Fermentationsversuch bei normaler Faulraumbelastung,
- Fig.9:: die Methangaskonzentration in einem kontinuierlichen Fermentationsversuch bei normaler Faulraumbelastung,
- Fig.10.: das Fettsäuremuster eines Substrats in einem kontinuierlichen Fermentationsversuch bei normaler Faulraumbelastung und
- Fig.11:: die Neutralisationskinetik der eingesetzten Additive.

In den Ausführungsbeispielen werden folgende kalkstämmige Materialien als Additive eingesetzt:
1. Biocal H (= KH, Kalkhydrat, Ca(OH)₂)
   - calcitisch
   - hohe Feinheit (d₅₀ = 7 µm, d₉₇ < 60 pm)
2. Biocal C (= KSM, Kalksteinmehl, CaCO₃
   - calcitisch
   - hohe Feinheit (d₅₀ = 9 µm, d₉₇ < 70 µm)
3. Biocal CM (= HB, Halbbrannt, CaCO₃^{∗}MgO)
   - dolomitisch
   - hohe Feinheit* (d₅₀ = 10 µm, d₉₇ < 90 µm)
   - Aktivierung durch Entsäuerung des Magnesiumanteils (Halbbrannt)
4. Bicar (= Natriumhydrogencarbonat, NaHCO₃)
5. Biocal SL (=Kalkmilch, Ca(OH)₂)
   - calcitisch
   - hohe Feinheit (d₅₀ = 2,5 µm, d₉₇ = 10 µm)
6. Biocal HM (=Dolomitkalkhydrat)
   - dolomitisch
   - hohe Feinheit (d₅₀ = 8 µm, d₉₇ = 50 µm)

### 1. Diskontinuierliche Fermentationsversuche

In den diskontinuierlichen Fermentationsversuchen wird die Wirksamkeit der Zugabe feinteiliger kalkstämmiger Materialien während der Fermentation organischer Substrate bei einmaliger Substratzufuhr untersucht.

Die diskontinuierlichen Versuche werden wie folgt durchgeführt:
Als Substrat wird Gülle eingesetzt. Das Substrat wird mittels Getreideschrot auf einen pH-Wert von ungefähr 6,3 angesäuert. Anschließend werden Maissilage sowie die untersuchten Additive zudosiert. Daraufhin wird über einen Zeitraum von 35 Tagen hinweg vergärt.

Die Dosierung der Additive kann stoffmengenbezogen oder pH-Wert-orientiert stattfinden. Unter einer stoffmengenbezogenen Dosierung wird in den vorliegenden Beispielen verstanden, dass jeweils die gleiche Stoffmenge an aktiven Ionen zugegeben wird (z.B. 0,012 mol/l Biocal H, 0,024 mol/l Biocal C). Unter einer pH-Wert-orientierten Dosierung wird verstanden, dass die Zugabe derart erfolgt, dass ein pH-Wert von 7,5 erreicht wird. Die unterschiedliche Lösekinetik kann hierbei zu einem anderen Verbrauch als bei der stoffmengenbezogenen Dosierung führen. Bei der pH-Wert-orientierten Dosierung wird das jeweilige Additiv jeweils bei Unterschreiten eines pH-Werts von 7,5 zugegeben.

Nach Beendigung des Versuchszeitraums von 35 Tagen werden folgende Parameter untersucht:
- pH-Wert
- Pufferkapazität
- Biogasproduktion
- Methangasproduktion.

### 1.1 Bestimmung der Pufferkapazität nach 35 Tagen Fermentation bei stoffmengenbezogener Additivdosierung

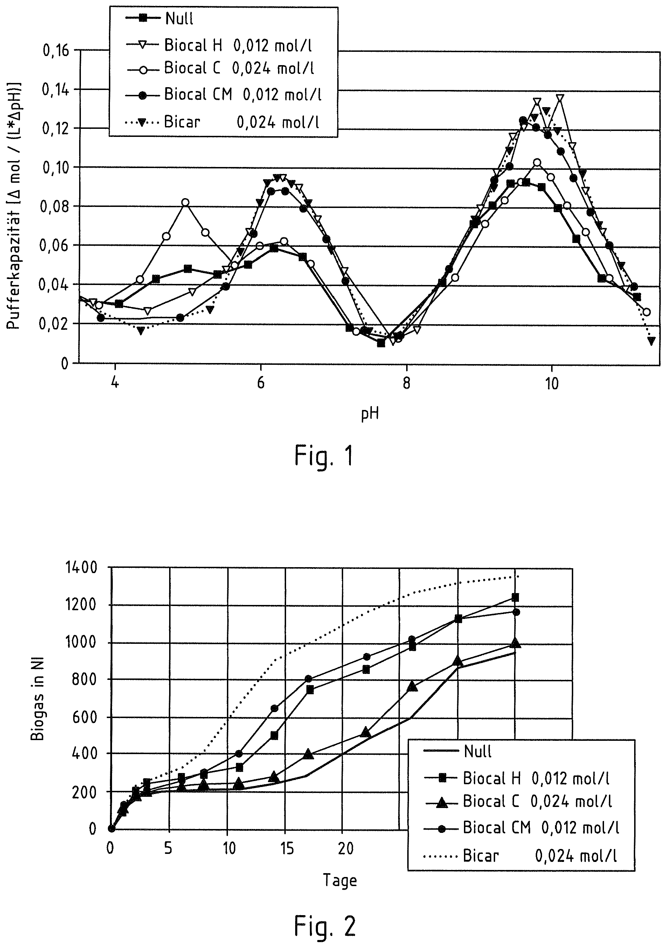

In Figur 1 ist die Pufferkapazität des Substrats in Abhängigkeit vom pH-Wert bei einer stoffmengenbezogenen Additivdosierung in Orientierung an Biocal H dargestellt. Wie aus Figur 1 hervorgeht, führt die Additivzugabe in allen Fällen zu einer erhöhten Pufferkapazität. Darüber hinaus zeigt sich, dass die Wirkung von Biocal H und Biocal CM sowie von Bicar bei einem pH-Wert von 6,5 am ausgeprägtesten ist.

### 1.2 Bestimmung der Biogasproduktion nach 35 Tagen Fermentation bei stoffmengenbezogener Additivdosierung

In Figur 2 ist die Biogasproduktion bei einer stoffmengenbezogenen Additivdosierung in Orientierung an Biocal H dargestellt. Aus dem Schaubild geht hervor, dass nach einer Versuchsdauer von 35 Tagen das Gesamtvolumen an Biogas bei der Verwendung der eingesetzten kalkstämmigen Materialien höher ausfällt.

### 1.3 Bestimmung der Methangasproduktion nach 35 Tagen Fermentation (stoffmengenbezogene Additivdosierung)

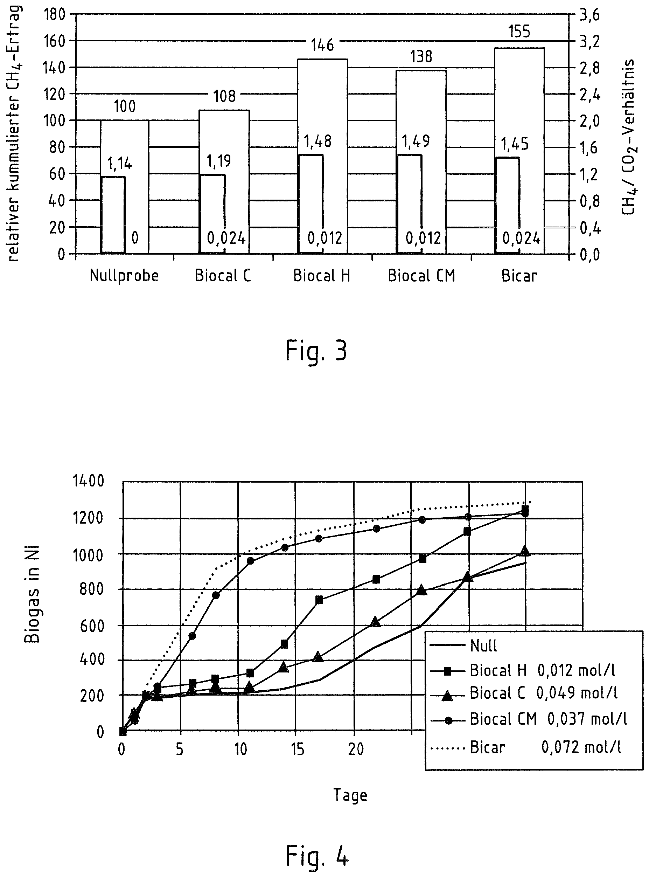

In Figur 3 ist die Methangasproduktion nach 35 Tagen bei einer stoffmengenbezogenen Additivdosierung in Orientierung an Biocal H dargestellt. Aus Figur 3 geht hervor, dass sich der Anteil an Methan im Biogas bei Verwendung der eingesetzten kalkstämmigen Materialien erhöht. Ferner zeigt sich, dass durch die Verwendung von Biocal CM das Verhältnis von Methan / Kohlendioxid in Biogas am stärksten erhöht wird.

### 1.4 Bestimmung der Biogasproduktion nach 35 Tagen Fermentation (pH-Wert-orientierte Additivdosierung)

In Figur 4 ist die Biogasproduktion nach 35 Tagen bei einer pH-Wert-orientierten Additivdosierung (max. pH = 7,5) dargestellt. Aus Figur 4 geht hervor, dass sich das Gesamtvolumen an Biogas bei Verwendung der eingesetzten kalkstämmigen Materialien erhöht.

### 1.5 Bestimmung der Methangasproduktion nach 35 Tagen Fermentation (pH-Wert-orientierte Additivdosierung)

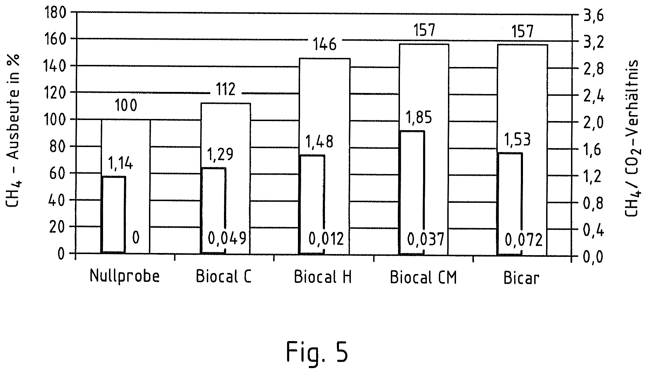

In Figur 5 ist die Methangasproduktion nach 35 Tagen bei einer pH-Wert-orientierten Additivdosierung (max. pH = 7,5) dargestellt. Die Zugabemenge von Biocal C, Biocal CM und Bicar wird in diesem Versuch verglichen mit dem in Figur 3 dargestellten Versuch erhöht. Aus Figur 5 geht hervor, dass die Methangasproduktion bei Verwendung einer höheren Additivdosierung weiter gesteigert werden kann.

### 1.6 Zusammenfassende Bewertung der diskontinuierlichen Versuchsreihe

- Die Pufferkapazität des mit den Additiven versetzten Fermenterinhaltes fällt höher aus als die Pufferkapazität des unmodifizierten Fermentinhalts.
- Nach einer Versuchsdauer von 35 Tagen fällt das Gesamtvolumen an Biogas und insbesondere an Methangas bei Verwendung der Additive höher aus.
- Durch pH-Wert-orientierte Zugabe von Additiven kann der Biogasertrag schon nach kürzester Zeit maximiert werden (schnellere Prozessregeneration).
- Es ist hervorzuheben, dass das Methan/Kohlendioxid-Verhältnis insbesondere bei Verwendung von Biocal CM im pH-Wert-orientierten Ansatz am Besten ausfällt (siehe Figuren 3/5).

### 2. Kontinuierliche Versuche

In diesen Fermentationsversuchen wird die Wirksamkeit der Zugabe von feinteiligen kalkstämmigen Materialien während der Fermentation organischer Substrate bei kontinuierlicher Prozessführung untersucht.

Die kontinuierlichen Versuche werden wie folgt durchgeführt:
Als Grundsubstrat wird Gülle eingesetzt. Es findet eine tägliche Fütterung mit Maissilage und Getreideschrot statt. Die Faulraumbelastung wird während der Versuchsreihe auf normale (3,5-4,5 g o TS/l/d) bis hohe Werte (9-12 g o TS/l/d) eingestellt. Die Additivzugabe findet pH-Wert-orientiert statt. Das heißt, die Additive werden zugegeben, wenn der pH-Wert im Fermenter einen minimalen pH-Wert von 6,3 unterschreitet. Die Zugabe erfolgt derart, dass ein pH-Wert von 7,5 eingestellt wird.

Folgende Parameter werden kontinuierlich gemessen:
- Gasquantität
- Gasqualität
- pH-Wert
- Pufferkapazität
- Fettsäuremuster

### 2.1 kontinuierliche Versuchsreihe (1)

In der kontinuierlichen Versuchsreihe (1) findet eine wiederholte Additivzugabe ab drohender Versauerung statt. Es wird eine gleichbleibend überhöhte Faulraumbelastung eingestellt.

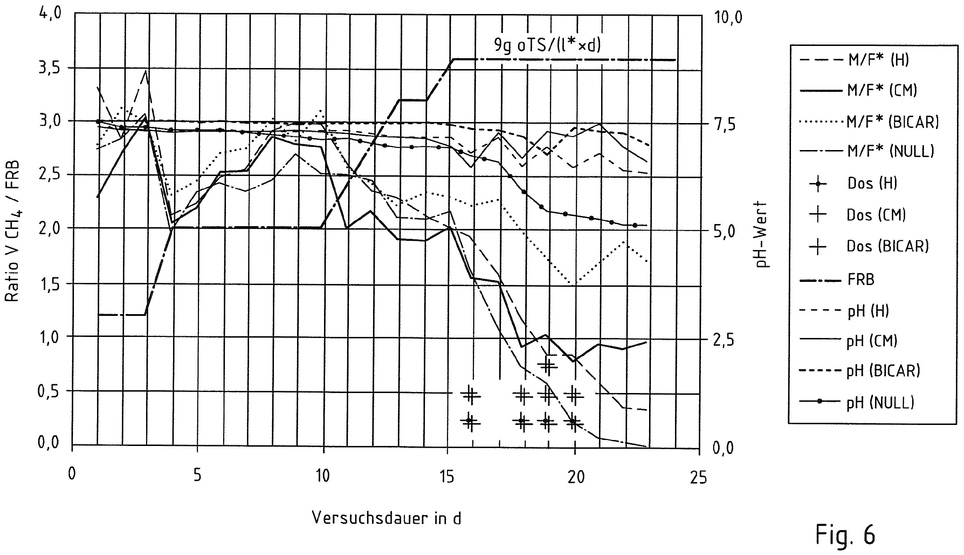

Wie in Figur 6 gezeigt, kommt der Fermentationsprozess bei Überfütterung und daraus resultierender Übersäuerung ohne Zugabe des Additivs zum Erliegen (siehe Nullprobe). Durch wiederholte Additivzugabe ab deutlich sinkendem pH-Wert und sinkender Pufferkapazität kann der Prozess jedoch dauerhaft stabilisiert werden. Die unterschiedliche Wirksamkeit der Additive lässt sich beispielsweise aus dem Verlauf der Kurve M/F (= Verhältnis von Methangasvolumen zu Faulraumbelastung) ableiten. Dieser Kurve lässt sich entnehmen, dass Biocal CM die besten Ergebnisse der kalkstämmigen Produkte zeigt.

### 2.2 kontinuierliche Versuchsreihe (2)

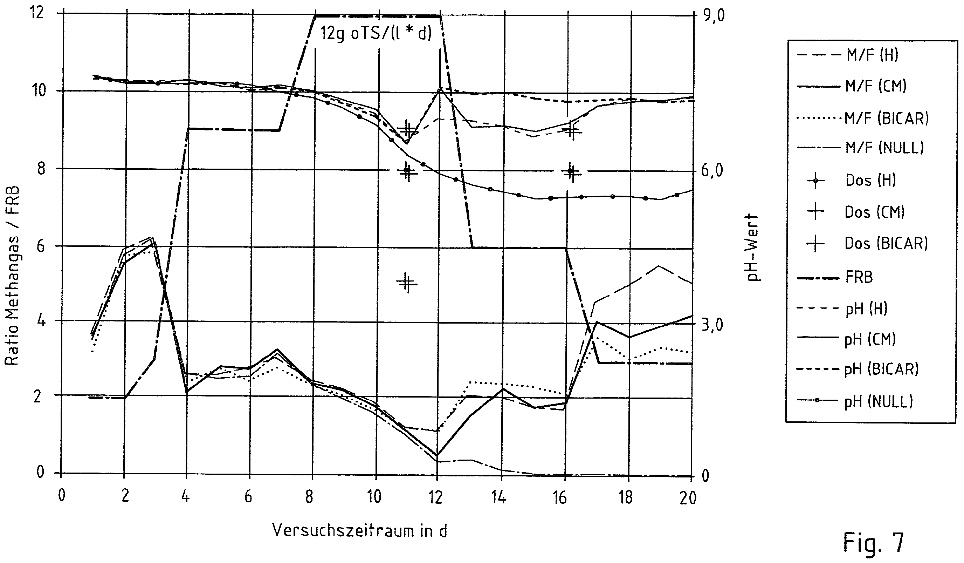

In Figur 7 ist die kontinuierliche Versuchsreihe (2) dargestellt. Die Prozessstabilisierung erfolgt in diesem Fall durch zweimalige Additivzugabe ab deutlich sinkendem pH-Wert und sinkender Pufferkapazität bei paralleler Reduzierung der Faulraumbelastung in zwei Stufen.

Aus Figur 7 geht hervor, dass ohne Additivzugabe der Fermentationsprozess bei fortfolgender Überfütterung und daraus resultierender Übersäuerung zum Erliegen kommt. Dieses Ergebnis entspricht dem in der kontinuierlichen Versuchsreihe (1) erzielten Ergebnis.

Es zeigt sich, dass bei dieser Versuchsreihe Biocal H gefolgt von Biocal CM die besten Ergebnisse zeigt.

### 2.3 kontinuierliche Versuchsreihe (3)

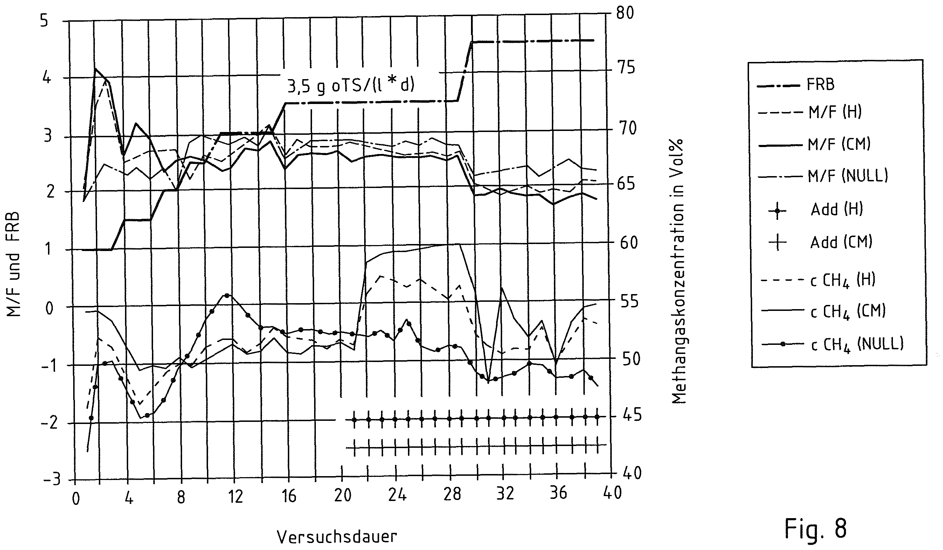

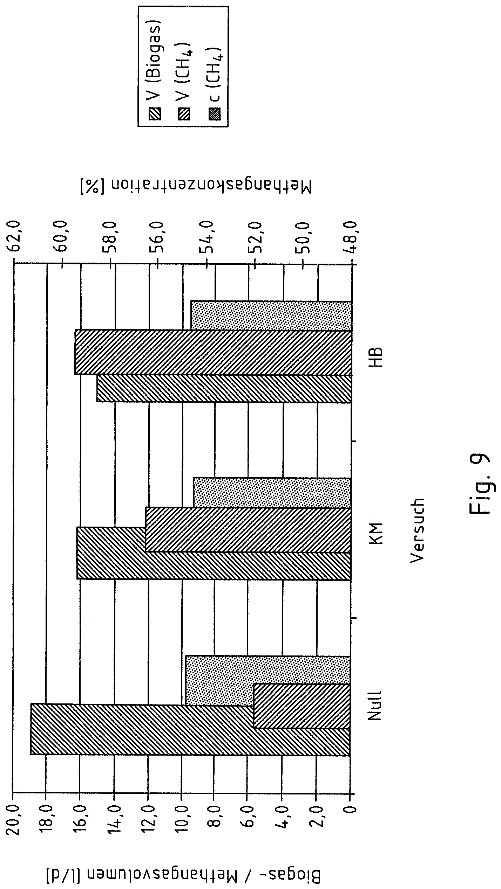

Figur 8 und Figur 9 zeigt die kontinuierliche Versuchsreihe (3). In dieser Versuchsreihe findet die Additivzugabe bei Normalbetrieb und normaler Faulraumbelastung statt.

Aus den Figuren 8 und 9 geht hervor, dass durch die Additivzugabe eine deutlich erhöhte Methangaskonzentration erzielt werden kann. Dieser Effekt ist bei der Verwendung von Biocal H und insbesondere von Biocal CM besonders deutlich. Die Reduzierung der CO₂-Konzentration im Biogas entspricht stöchiometrisch der zugesetzten Additivmenge
Δ V CO₂ = 0,011 mol/l bei 0,01 mol/l Dosiermenge Biocal H
Δ V CO₂ = 0,015 mol/l bei 0,01 mol/l Dosiermenge Biocal CM.

### 2.4 kontinuierliche Versuchsreihe Fettsäuremuster

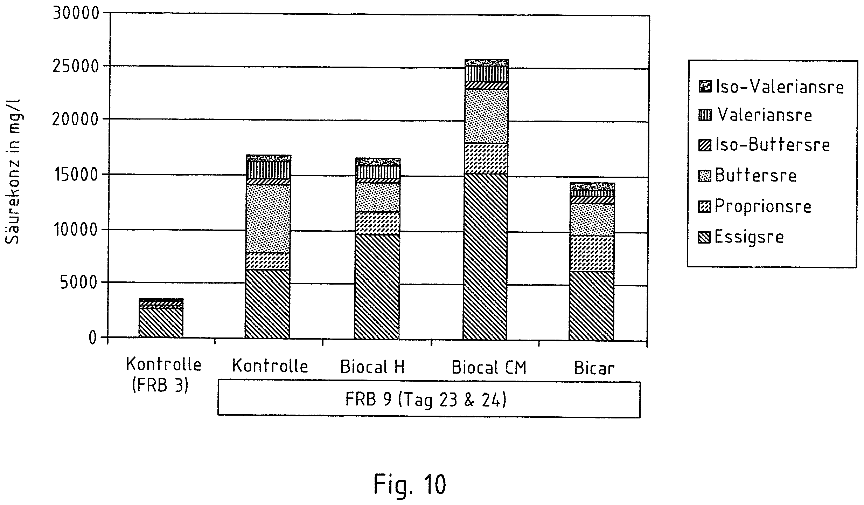

In Figur 10 ist dargestellt, wie sich bei der kontinuierlichen Versuchsreihe das Fettsäuremuster der in den Versuchen eingesetzten organischen Materialien verändert. Untersucht wird die Veränderung bei hoher und normaler Faulraumbelastung.

Bei einer hohen Faulraumbelastung zeigt sich, dass die Überfütterung eine Fettsäureakkumulation erzeugt. Dies ist ein Indiz für eine Abbauhemmung während des Fermentationsvorgangs. Darüber hinaus geht aus Figur 10 hervor, dass sich die erzielten Fettsäuremuster in Abhängigkeit von dem jeweils eingesetzten Additiv unterscheiden. Mit Biocal CM wird die höchste Gesamtsäurekonzentration (Faktor 7) und Essigsäurekonzentration (Faktor 6) im Vergleich zur Ausgangssituation erzielt. Das Essigsäure/Propionsäure-Verhältnis halbiert sich bei Einsatz von Biocal H und Biocal CM im Vergleich zur Ausgangssituation (Verhältniswert = 9).

Bei normaler Faulraumbelastung wird im stabilen Betrieb durch den Einsatz von Biocal H oder Biocal CM im Vergleich zur Nullprobe die Gesamtfettsäurekonzentration erhöht. Die Fettsäuremuster unterscheiden sich von der Nullprobe.

### 2.5 kontinuierliche Versuchsreihe Neutralisationskinetik

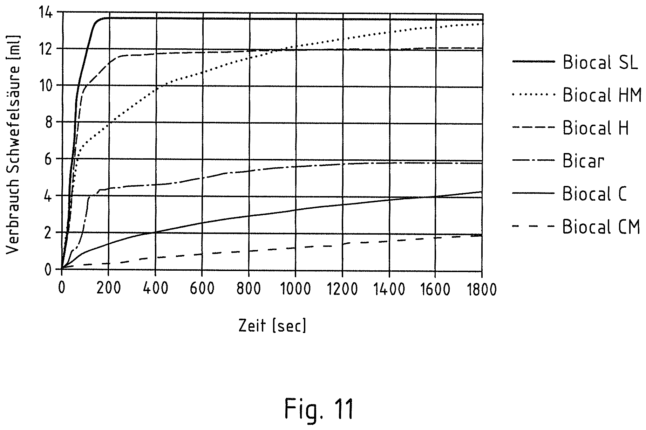

In Figur 11 ist die Neutralisationskinetik der verschiedenen untersuchten Additive angezeigt. Die Bandbreite der produktspezifischen Reaktionskinetik wird durch Titration mit Schwefelsäure auf den pH-Wert von 6 bestimmt. Aus Figur 11 geht hervor, dass Biocal H sehr spontan reagiert. Dagegen reagiert Biocal CM mit einer gewissen Verzögerung.

### 2.6 Zusammenfassende Bewertung

Zusammenfassend konnte gezeigt werden, dass mit einer gezielten Zugabe feinteiliger kalkstämmiger Materialien eine schnelle Prozessregeneration bei Substratversauerung erzielt werden kann. Dies ist insbesondere bei Stoßbelastungen beispielsweise durch die verstärkte Nutzung landwirtschaftlicher Abfälle hilfreich. Insbesondere wurde festgestellt, dass feinteilige kalkstämmige Materialien keine bzw. nur geringe Sedimentationsneigung im Fermenter zeigen. Somit kann in dem erfindungsgemäßen Verfahren der pH-Wert während des Fermentationsprozesses zuverlässig stabilisiert werden. Dies ermöglicht den Einsatz hochenergetischer Substrate bei gleichzeitig hoher Raumbelastung.

Bei permanentem Einsatz zeigen die eingesetzten kalkstämmigen Materialien den Vorteil die Pufferkapazität im Bioreaktor zu erhöhen. Somit wirken sie vorbeugend gegen eine Versauerung.

Darüber hinaus wurde festgestellt, dass die Zugabe dieser Materialien zu einer Erhöhung bzw. einer Veränderung der Fettsäuren im Substrat führt.

Ein weiterer überraschender Vorteil des Einsatzes feinteiliger kalkstämmiger Materialien besteht darin die Methangaskonzentration im Biogas zu steigern. Nach derzeitigem Kenntnisstand findet die Erhöhung der Methangaskonzentration durch in-situ-Abscheidung von Kohlendioxid (CO₂) statt. Eine hohe Methangaskonzentration ist insbesondere bei der Biogasaufbereitung für die Direkteinspeisung in Erdgasnetze von Vorteil. Darüber hinaus wird die Effizienz bei der Verwertung in Blockheizkraftwerken gesteigert.

Sowohl im permanenten als auch im sporadischen Einsatz zeichnen sich die kalkstämmigen Materialien durch eine hygienisierende Wirkung aus. Dies ist insbesondere bei der Verwendung von landwirtschaftlichen Abfällen als Substrat von Vorteil. Zudem wird der Gärrest durch Nährsstoffe, die aus den kalkstämmigen Materialien stammen, angereichert, was bei der Verwendung der Gärreste als biologischer Dünger sinnvoll sein kann. Für die Pflanzenernährung sind in diesem Zusammenhang insbesondere Calcium und Magnesium relevant.

## Patentansprüche

1. Verfahren zur Erzeugung von Biogas aus einem biologisch abbaubaren Substrat unter im Wesentlichen anaeroben Bedingungen, umfassend folgende Schritte:
- Einbringen des Substrats in einen Reaktionsraum,
- Zugabe eines kalkstämmigen Materials zu dem Substrat,
- biologischer Abbau des Substrats unter Bildung von Biogas, wobei
ein kalkstämmiges Material, ausgewählt aus der Gruppe bestehend aus Kalkhydrat, Kalkmilch, Kalksteinmehl, Branntkalk, Dolomitsteinmehl, Dolomithalbbrannt, Dolomitkalkhydrat, Dolomithalbhydrat und/oder Natriumhydrogencarbonat eingesetzt wird, **dadurch gekennzeichnet, dass** das kalkstämmige Material einen Teilchendurchmesser d₉₇ von 1 bis 70 µm und/oder einen Teilchendurchmesser d₅₀ von 1 bis 8 µm aufweist.

2. Verfahren nach Anspruch 1, **dadurch gekennzeichnet, dass** ein kalkstämmigen Material eingesetzt wird, das einen Teilchendurchmesser d₉₇ von weniger als 50 µm, vorzugsweise von weniger als 25 µm und insbesondere von weniger als 15 µm aufweist.

3. Verfahren nach einem der Ansprüche 1 oder 2, **dadurch gekennzeichnet, dass** die Zugabe des kalkstämmigen Materials dann vorgenommen wird, wenn der pH-Wert des Substrats unter einen Wert von 7,5 fällt.

4. Verfahren nach einem der Ansprüche 1 oder 2, **dadurch gekennzeichnet, dass** die Zugabe des kalkstämmigen Materials präventiv zur Vorbeugung einer Substratversauerung erfolgt.

5. Verfahren nach einem der Ansprüche 1 bis 4, **dadurch gekennzeichnet, dass** das kalkstämmige Material in einer Menge von 0,0001 bis 0,05 mol/l, vorzugsweise von 0,001 bis 0,02 mol/l, und insbesondere von 0,005 bis 0,01 mol/l, jeweils bezogen auf das Fermentervolumen, zugegeben wird.

6. Verfahren nach einem der Ansprüche 1 bis 5, **dadurch gekennzeichnet, dass** das kalkstämmige Material in einer solchen Menge zugegeben wird, dass sich die Pufferkapazität, ausgedrückt als Konzentration CaCO₃ pro Liter Fermentervolumen, auf einen Wert von mehr als 10 g/l, vorzugweise mehr als 15 g/l, und insbesondere, von 15 bis 20 g/l einstellt.

7. Verfahren nach einem der Ansprüche 1 bis 6, **dadurch gekennzeichnet, dass** der biologische Abbau folgende Schritte umfasst:
- Aufspaltung von im Substrat enthaltenen Kohlenhydraten, Fetten und/oder Eiweißen zu Spaltprodukten umfassend Zucker, Fettsäuren, Aminosäuren und/oder Basen,
- Fermentation der Spaltprodukte zu Vergärungsprodukten umfassend Carbonsäuren, Gase und/oder Alkohole,
- Bildung von methanogenen Substraten umfassend Essigsäure, Wasserstoff und/oder Kohlendioxid,
- Bildung von Biogas umfassend Methan und Kohlendioxid.

8. Verfahren nach Anspruch 7, **dadurch gekennzeichnet, dass** die Aufspaltung von im Substrat enthaltenen Kohlenhydraten, Fetten und/oder Eiweißen durch hydrolytische Bakterien bewirkt wird.

9. Verfahren nach Anspruch 7 oder 8, **dadurch gekennzeichnet, dass** die Vergärung der Spaltprodukte zu Vergärungsprodukten durch fermentative Bakterien bewirkt wird.

10. Verfahren nach einem der Ansprüche 7 bis 9, **dadurch gekennzeichnet, dass** die Bildung von methanogenen Substraten durch acetogene Bakterien bewirkt wird.

11. Verfahren nach einem der Ansprüche 7 bis 10, **dadurch gekennzeichnet, dass** die Bildung von Biogas durch methanogene Bakterien bewirkt wird.

12. Verfahren nach einem der Ansprüche 7 bis 11, **dadurch gekennzeichnet, dass** mindestens zwei Schritte des biologischen Abbaus in demselben Reaktionsraum stattfinden.

13. Verfahren nach einem der Ansprüche 1 bis 12, **dadurch gekennzeichnet, dass** ein Substrat mit einem C:N:P:S-Verhältnis von etwa 600 : 15 : 5 : 1 eingesetzt wird.

14. Verfahren nach einem der Ansprüche 1 bis 13, **dadurch gekennzeichnet, dass** ein Substrat mit einem C:N-Verhältnis von 10 bis 30 eingesetzt wird.

15. Verfahren nach einem der Ansprüche 1 bis 14, **dadurch gekennzeichnet, dass** die Reaktionstemperatur im Fermenter auf einen Wert von 28 bis 46°C eingestellt wird.

16. Verfahren nach einem der Ansprüche 1 bis 15, **dadurch gekennzeichnet, dass** die Raumbelastung, gemessen als zugesetzte Masse an organischer Trockensubstanz der eingesetzten Substrate pro Fermentervolumen- und Zeiteinheit, auf einen Wert von 0,5 bis 5 kg oTS/m³ Tag, vorzugsweise 1 bis 3 kg oTS/m³ Tag eingestellt wird.

17. Verfahren nach einem der Ansprüche 1 bis 16, **dadurch gekennzeichnet, dass** Biogas, enthaltend mehr als 68 Vol.% Methan hergestellt wird.

18. Verwendung eines kalkstämmigen Materials, ausgewählt aus der Gruppe bestehend aus Kalkhydrat, Kalkmilch, Kalksteinmehl, Branntkalk, Dolomitsteinmehl, Dolomithalbbrannt, Dolomitkalkhydrat, Dolomithalbhydrat und/oder Natriumhydrogencarbonat mit einem Teilchendurchmesser d₉₇ von 1 bis 70 µm und/oder mit einem Teilchendurchmesser d₅₀ von 1 bis 8 µm als Additiv zur Regulierung des pH-Werts eines biologisch abbaubaren Substrats bei der Erzeugung von Biogas.

## Claims

1. Method for producing biogas from a biodegradable substrate under substantially anaerobic conditions, comprising the following steps:
- introducing the substrate into a reaction chamber,
- adding a lime-based material to the substrate,
- biodegrading of the substrate to form biogas,
wherein a lime-based material selected from the group consisting of hydrated lime, milk of lime, limestone powder, burnt lime, dolomite powder, half-burnt dolomite, hydrated dolomitic lime, semi-hydrated dolime and/or sodium hydrogen carbonate is used, **characterized in that** the lime-based material has a particle diameter d₉₇ of 1 to 70 µm and/or a particle diameter d₅₀ of 1 to 8 µm.

2. Method according to claim 1, **characterized in that** a lime-based material is used which has a particle diameter d₉₇ of less than 50 µm, preferably less than 25 µm, and in particular less than 15 µm.

3. Method according to either claim 1 or claim 2, **characterized in that** the lime-based material is added when the pH of the substrate falls below 7.5.

4. Method according to either claim 1 or claim 2, **characterized in that** the lime-based material is added as a preventative measure to prevent substrate acidification.

5. Method according to any of claims 1 to 4, **characterized in that** the lime-based material is added in an amount of from 0.0001 to 0.05 mol/l, preferably from 0.001 to 0.02 mol/l, and in particular from 0.005 to 0.01 mol/l, in each case based on the fermenter volume.

6. Method according to any of claims 1 to 5, **characterized in that** the lime-based material is added in such an amount that the buffer capacity, expressed as a concentration CaCO₃ per liter fermenter volume, is adjusted to a value of more than 10 g/l, preferably more than 15 g/l, and, in particular, from 15 to 20 g/l.

7. Method according to any of claims 1 to 6, **characterized in that** the biodegradation comprises the following steps:
- splitting carbohydrates, fats and/or proteins contained in the substrate to form decomposition products comprising sugars, fatty acids, amino acids and/or bases,
- fermenting the decomposition products to form fermentation products comprising carboxylic acids, gases and/or alcohols,
- forming methanogenic substrates comprising acetic acid, hydrogen and/or carbon dioxide,
- forming biogas comprising methane and carbon dioxide.

8. Method according to claim 7, **characterized in that** the splitting of carbohydrates, fats and/or proteins contained in the substrate is caused by hydrolytic bacteria.

9. Method according to either claim 7 or claim 8, **characterized in that** the fermentation of the decomposition products to form fermentation products is caused by fermentative bacteria.

10. Method according to any of claims 7 to 9, **characterized in that** the formation of methanogenic substrates is caused by acetogenic bacteria.

11. Method according to any of claims 7 to 10, **characterized in that** the formation of biogas is caused by methanogenic bacteria.

12. Method according to any of claims 7 to 11, **characterized in that** at least two steps of the biodegradation take place in the same reaction chamber.

13. Method according to any of claims 1 to 12, **characterized in that** a substrate having a C:N:P:S ratio of approximately 600:15:5:1 is used.

14. Method according to any of claims 1 to 13, **characterized in that** a substrate having a C:N ratio of from 10 to 30 is used.

15. Method according to any of claims 1 to 14, **characterized in that** the reaction temperature in the fermenter is adjusted to a value of from 28 to 46°C.

16. Method according to any of claims 1 to 15, **characterized in that** the volumetric loading, measured as added mass of organic dry matter of the substrates used per fermenter volume and time unit, is adjusted to a value of from 0.5 to 5 kg oTS/m³ per day, preferably 1 to 3 kg oTS/m³ per day.

17. Method according to any of claims 1 to 16, **characterized in that** biogas containing more than 68 vol.% methane is produced.

18. Use of a lime-based material selected from the group consisting of hydrated lime, milk of lime, limestone powder, burnt lime, dolomite powder, half-burnt dolomite, hydrated dolomitic lime, semi-hydrated dolime and/or sodium hydrogen carbonate, having a particle diameter d₉₇ of 1 to 70 µm and/or a particle diameter d₅₀ of 1 to 8 µm, as an additive for regulating the pH of a biodegradable substrate in the production of biogas.

## Revendications

1. Procédé de production de biogaz à partir d'un substrat biologiquement dégradable dans des conditions pratiquement anaérobies, comprenant les étapes suivantes :
- introduction du substrat dans un espace de réaction,
- ajout d'un matériau calcique au substrat,
- dégradation biologique du substrat pour former du biogaz,
dans lequel l'on utilise un matériau calcique choisi dans le groupe constitué de la chaux hydratée, du lait de chaux, de la poudre de calcaire, de la chaux vive, de la poudre de dolomie, de la dolomie semi-calcinée, de la chaux hydratée dolomitique, de la dolomie semi-hydratée et/ou de l'hydrogénocarbonate de sodium, **caractérisé en ce que** le matériau calcique présente un diamètre de particule d₉₇ de 1 à 70 µm et/ou un diamètre de particule d₅₀ de 1 à 8 µm.

2. Procédé selon la revendication 1, **caractérisé en ce qu'**un matériau calcique est utilisé, lequel présente un diamètre de particule d₉₇ inférieur à 50 µm, de préférence inférieur à 25 µm et en particulier inférieur 15 µm.

3. Procédé selon l'une des revendications 1 ou 2, **caractérisé en ce que** l'ajout du matériau calcique est effectué lorsque le pH du substrat tombe en dessous d'une valeur de 7,5.

4. Procédé selon l'une des revendications 1 ou 2, **caractérisé en ce que** l'ajout du matériau calcique a lieu de manière préventive pour empêcher une acidification du substrat.

5. Procédé selon l'une des revendications 1 à 4, **caractérisé en ce que** le matériau calcique est ajouté en une quantité de 0,0001 à 0,05 mol/l, de préférence de 0,001 à 0,02 mol/l, et en particulier de 0,005 à 0,01 mol/l, dans chaque cas par rapport au volume de fermenteur.

6. Procédé selon l'une des revendications 1 à 5, **caractérisé en ce que** le matériau calcique est ajouté en une quantité telle que la capacité du tampon, exprimée en termes de concentration de CaCO₃ par litre de volume de fermenteur, est ajusté à une valeur supérieure à 10 g/l, de préférence supérieure à 15 g/l, et en particulier de 15 à 20 g/l.

7. Procédé selon l'une des revendications 1 à 6, **caractérisé en ce que** la dégradation biologique comprend les étapes suivantes :
- fractionnement d'hydrates de carbone, de graisses et/ou de protéines contenus dans le substrat en produits de décomposition comprenant des sucres, des acides gras, des acides aminés et/ou des bases,
- fermentation de produits de décomposition en produits de fermentation, comprenant des acides carboxyliques, des gaz et/ou des alcools,
- formation de substrats méthanogènes comprenant de l'acide acétique, de l'hydrogène et/ou du dioxyde de carbone,
- formation de biogaz comprenant du méthane et du dioxyde de carbone.

8. Procédé selon la revendication 7, **caractérisé en ce que** le fractionnement des hydrates de carbone, des graisses et/ou des protéines contenus dans le substrat est provoqué par des bactéries hydrolytiques.

9. Procédé selon la revendication 7 ou 8, **caractérisé en ce que** la fermentation des produits de décomposition en produits de fermentation est provoquée par des bactéries fermentatives.

10. Procédé selon l'une des revendications 7 à 9, **caractérisé en ce que** la formation de substrats méthanogènes est provoquée par des bactéries acétogènes.

11. Procédé selon l'une des revendications 7 à 10, **caractérisé en ce que** la formation de biogaz est provoquée par des bactéries méthanogènes.

12. Procédé selon l'une des revendications 7 à 11, **caractérisé en ce qu'**au moins deux étapes de la dégradation biologique ont lieu dans la même chambre de réaction.

13. Procédé selon l'une des revendications 1 à 12, **caractérisé en ce qu'**un substrat présentant un rapport C:N:P:S d'environ 600:15:5:1 est utilisé.

14. Procédé selon l'une des revendications 1 à 13, **caractérisé en ce qu'**un substrat présentant un rapport C:N de 10 à 30 est utilisé.

15. Procédé selon l'une des revendications 1 à 14, **caractérisé en ce que** la température de réaction est réglée dans le fermenteur à une valeur de 28 à 46 °C.

16. Procédé selon l'une des revendications 1 à 15, **caractérisé en ce que** la charge volumique, exprimée en masse ajoutée de matière sèche organique des substrats utilisés par volume de fermenteur et unité de temps, est réglée à une valeur de 0,5 à 5 kg oTS/m³ par jour, de préférence de 1 à 3 kg oTS/m³ par jour.

17. Procédé selon l'une des revendications 1 à 16, **caractérisé en ce que** un biogaz, contenant plus de 68 % en volume de méthane est produit.

18. Utilisation d'un matériau calcique choisi dans le groupe constitué de la chaux hydratée, du lait de chaux, de la poudre de calcaire, de la chaux vive, de la poudre de dolomie, de la dolomie semi-calcinée, de la chaux hydratée dolomitique, de la dolomie semi-hydratée et/ou de l'hydrogénocarbonate de sodium présentant un diamètre de particule d₉₇ de 1 à 70 µm et/ou un diamètre de particule d₅₀ de 1 à 8 µm comme additif pour la régulation du pH d'un substrat biologiquement dégradable dans la production de biogaz.
